# EUROPÄISCHE PATENTSCHRIFT

(11) **EP 0 575 290 B1**
(45) Veröffentlichungstag und Bekanntmachung des Hinweises auf die Patenterteilung: **23.08.2006**
(21) Anmeldenummer: 93810410.6
(22) Anmeldetag: 08.06.1993
(51) Int. Cl.: A61L 2/18, A01N 47/44, G02C 13/00

(54) **Kontaktlinsenpflegemittel für harte und weiche Kontaktlinsen**
Product for care of hard or soft contact lenses
Produit pour l'entretien des lentilles de contact dures ou molles

(30) Priorität: 17.06.1992 EP 92810467
(43) Veröffentlichungstag der Anmeldung: 22.12.1993
(73) Patentinhaber: Novartis AG, 4056 Basel (CH); Novartis Pharma GmbH, 1230 Wien (AT)
(72) Erfinder: Schwind, Peter, W-8759 Hösbach-Rottenberg (DE)

(56) Entgegenhaltungen:
- WO-A-91/17469
- WO-A-92/11876
- AU-B- 565 948
- US-A- 4 490 389

## Beschreibung

Die vorliegende Erfindung betrifft ein Kontaktlinsenpflegemittel für harte und weiche Kontaktlinsen enthaltend ein polymeres Biguanid und einen speziellen Puffer.

Kontaktlinsenpflegemittel enthaltend ein Biguanid sind bereits bekannt. So werden in der GB 1,432,345 ophthalmische Zusammensetzungen und Kontaktlinsen desinfizierende Zusammensetzungen beschrieben, die ein ophthalmologisch annehmbares polymeres Biguanid enthalten. Als in diesem Zusammenhang geeignete Puffer werden ausschliesslich Phosphat-Puffer offenbart.

Auch in der EP-A1-180,309 werden Desinfektions- und Konservierungslösungen für Kontaktlinsen offenbart, die ein Biguanid und einen Puffer enthalten. Als Puffer werden insbesondere Borat-Puffer vorgeschlagen. Zusätzlich als Puffer genannt sind Citrat-Puffer, Bicarbonat-Puffer und gemischte Phosphat-Puffer.

Demgegenüber bezieht sich die vorliegende Erfindung auf Kontaktlinsenpflegemittel enthaltend ein Biguanid oder ein Salz davon und als Puffer den sogenannten Tris-Puffer (Trometamol) oder ein Homologes davon mit bis zu 10 Kohlenstoffatomen oder ein Salz davon. Die Erfindung bezieht sich ebenfalls auf die Verwendung eines solchen Kontaktlinsenpflegemittels zur Reinigung und Desinfektion von Kontaktlinsen.

Die erfindungsgemäss zu verwendenden Biguanide weisen die Formel I auf, 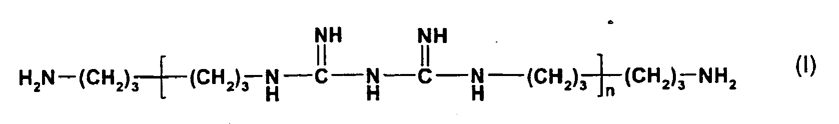 worin n eine ganze Zahl zwischen 1 und 500 bedeutet, ferner können auch Salze von Verbindungen der Formel I verwendet werden, insbesondere ophthalmologisch annehmbare Salze davon.

Die Verbindungen der Formel I sind bekannt. Ihre Herstellung ist z.B. in den GB 702,268 und GB 1,152,243 beschrieben. Darüberhinaus sind diese Verbindungen auch im Handel erhältlich, z.B. als Vantocil® , Cosmocil® oder als Arlagard® E von ICI Chemicals.

Die Verbindungen der Formel I können, je nach ihrer Herstellungsweise gewisse Anteile eines Nebenproduktes der Formel II enthalten, 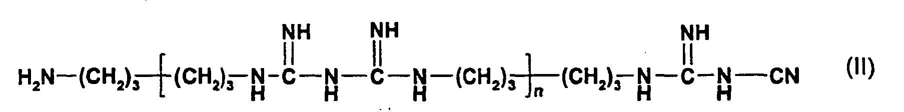 oder Salze davon, worin ebenfalls n eine ganze Zahl zwischen 1 und 500 bedeutet. Gemische von Verbindungen der Formel I mit solchen der Formel II können erfindungsgemäss ebenfalls verwendet werden. Der Anteil von Verbindungen der Formel II, bezogen auf die Gesamtmenge von Verbindungen der Formel I und Verbindungen der Formel II, liegt vorzugsweise bei weniger als 20 Gewichtsprozent, stärker bevorzugt bei weniger als 2 bis 10 Gewichtsprozent und beträgt besonders bevorzugt Null Gewichtsprozent.

Der Index n in den Formeln I respektive II bedeutet vorzugsweise 1 bis 200, insbesondere 2 bis 100, besonders bevorzugt 2 bis 50 und ganz besonders bevorzugt 3 bis 12. Je nach Bedeutung des Index n in den Formeln I oder II liegt das Molekulargewicht der verwendbaren Biguanide so niedrig wie dasjenige Molekulargewicht des Monomeren der Formel I (n = 1), oder im Bereich von etwa 600 bis 1600, falls Oligomere verwendet werden, d.h. wenn n beispielsweise für 3 bis 8 steht, oder auch im Bereich von etwa 50 000 bis etwa 90 000, falls n für deutliche höhere Werte steht, z.B. für etwa 270 bis 500.

Die Verbindung der Formel I wird in den erfindungsgemässen Kontaktlinsenpflegemitteln vorzugsweise in einer Menge, bezogen auf die Gesamtmenge des vorteilhafterweise in wässriger Lösung formulierten Kontaktlinsenpflegemittels, von 0,1 bis 100 ppm (0,00001 - 0,01 Gewichtsprozent) verwendet, insbesondere in einer Menge von 0,5 bis 50 ppm (0,00005 - 0,005 Gewichtsprozent) und besonders bevorzugt in einer Menge von 1 bis 10 ppm (0,0001 - 0,001 Gewichtsprozent), z.B. 1, 2 oder 5 ppm, verwendet.

Die im Rahmen der vorliegenden Erfindung geeigneten Salze von Verbindungen der Formel I respektive der Formel 11 sind wasserlösliche Salze, die vorteilhafterweise ophthalmologisch annehmbar sind. Geeignete Salze sind solche mit anorganischen oder organischen Säuren, beispielsweise Hydrochloride, Hydrobromide, Borate, Acetate, Gluconate, Sulfonate, Maleate, Ascorbate, Tartrate oder Citrate.

Bei dem erfindungsgemäss zur Anwendung kommenden Trometamol und dessen Homologen mit bis zu 10, bevorzugt mit 5 bis 7 Kohlenstoffatomen, handelt es sich um die Verbindungen der Formel III, 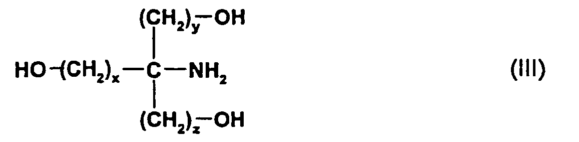 worin x, y und z, unabhängig voneinander, eine ganze Zahl, mindestens 1, bedeuten und die Summe aus x, y und z 3 bis 9 beträgt, bevorzugt 3 bis 6, oder ein Salz davon. In erster Linie bevorzugt ist 2-Amino-2-hydroxymethyl-1,3-propandiol (Trometamol), das einer Verbindung der Formel III entspricht, worin x und y und z jeweils 1 bedeuten. Die Verbindung Trometamol wird auch als Tris-Puffer bezeichnet.

Trometamol und dessen Homologe mit bis zu 10 Kohlenstoffatomen sind bereits bekannt. Ihre Anwendung in Arzneimitteln zur Behandlung von Entzündungen im Auge wurde bereits in der EP-A2-242,328 offenbart. Aus der WO 92/11876 sind bereits Zusammensetzungen für die Desinfektion von Kontaktlinsen bekannt, die 1,2 % Tromethamin / Tromethamin-hydrochlorid und 1 ppm Polyhexamethylenbiguanidhydrochlorid enthalten. Im übrigen sind auch Verbindungen der Formel III im Handel erhältlich.

Trometamol wird als Puffer in den erfindungsgemässen Kontaktlinsenpflegemitteln in einer Menge, bezogen auf die Gesamtmenge des Kontaktlinsenpflegemittels, von 0,05 bis weniger als 0,6 Gewichtsprozent verwendet oder insbesondere in einer Menge von 0,1 bis weniger als 0,6 Gewichtsprozent, wie z.B. bis zu 0,5 Gewichtsprozent.

Als Puffer kann 2-Amino-2-hydroxymethyl-1,3-propandiol oder ein Homologes davon mit bis zu 10 Kohlenstoffatomen, oder ein Salz davon, allein verwendet werden. Alternativ dazu kann eine der vorgenannten Verbindungen zusammen mit einem Salz davon verwendet werden. Wiederum werden hier vorzugsweise ophthalmologisch annehmbare Salze verwendet, wie z.B. die vorstehend im Zusammenhang mit Verbindungen der Formel I genannten Salze. Besonders geeignet sind die Hydrochloride oder Maleate von Verbindungen der Formel III, also beispielsweise die Kombination von 2-Amino-2-hydroxymethyl-1,3-propandiol mit dem Hydrochlorid von 2-Amino-2-hydroxymethyl-1,3-propandiol, ebenfalls die Kombination von 2-Amino-2-hydroxymethyl-1,3-propandiol mit dem Maleat von 2-Amino-2-hydroxymethyl-1,3-propandiol oder 2-Amino-2-hydroxymethyl-1,3-propandiol, dem geringe Mengen von Salzsäure zugesetzt werden.

Andere Puffer als Verbindungen der Formel III sind in den erfindungsgemässen Kontaktlinsenpflegemitteln vorzugsweise nicht enthalten. Sie können jedoch neben einer Verbindung oder mehrerer Verbindungen der Formel III zusätzlich enthalten sein.

Die erfindungsgemässen Kontaktlinsenpflegemittel werden vorzugsweise so formuliert, dass sie mit der Tränenflüssigkeit isotonisch sind. Sie können allgemein Zusatzstoffe enthalten, wie sie für Kontaktlinsenpflegemittel üblich sind. Darunter fallen z.B. die Tonizität beeinflussende Substanzen, oberflächenaktive Substanzen, die Viskosität beeinflussende Substanzen, oder Komplexbildner. Die Mengen, in denen diese oder weitere übliche Zusatzstoffe in den erfindungsgemässen Kontaktlinsenpflegemitteln enthalten sind, bewegen sich im Rahmen der dem Fachmann geläufigen Werte.

Unter einer Lösung, die mit der Tränenflüssigkeit isoton ist, wird allgemein eine Lösung verstanden, deren Konzentration der Konzentration einer 0,9 %-igen Kochsalzlösung entspricht. Abweichungen hiervon sind durchaus möglich, solange die zu behandelnden Kontaktlinsen dabei nicht geschädigt werden. Die Isotonie mit der Tränenflüssigkeit oder auch eine andere gewünschte Tonizität kann durch Zugabe von organischen oder anorganischen die Tonizität beeinflussenden Substanzen eingestellt werden. Erstere können z.B. in Mengen von etwa 1 bis 4,5 Gewichtsprozent verwendet werden, letztere in Mengen von etwa 0,1 bis 1,3 Gewichtsprozent. Generell wird soviel der die Tonizität beeinflussenden Substanz zugegeben, dass die Tonizität der erfindungsgemässen Zusammensetzung insbesondere im Bereich von 200 bis 450 Milliosmol liegt, vorzugsweise im Bereich von etwa 270 bis etwa 330 Milliosmol. Typische organische Substanzen dieser Art sind beispielsweise Glycerin, Harnstoff, Propylenglykol oder Zucker wie Mannit oder Sorbit, typische anorganische Substanzen dieser Art sind insbesondere Kaliumchlorid oder Natriumchlorid. Auch Gemische dieser Verbindungen miteinander können erfindungsgemäss verwendet werden.

Geeignete oberflächenaktive Substanzen sind beispielsweise in der EP-A2-180,309 genannt. Als besonders geeignete Vertreter, die erfindungsgemäss verwendet werden können, seien hier beispielsweise Poloxamer-Typen oder Miranol-Typen genannt. Weitere Vertreter sind dem Fachmann bekannt. Diese Substanzen können beispielsweise in Mengen von bis zu 20 Gewichtsprozent, insbesondere in Mengen von 0,4 bis 5 Gewichtsprozent verwendet werden.

Geeignete die Viskosität beeinflussende Substanzen sind dem Fachmann ebenfalls bekannt. Als besonders geeignete Vertreter, die erfindungsgemäss verwendet werden können, seien hier beispielsweise Polyvinylalkohol, Hydroxyethylcellulose, Hydroxypropylmethylcellulose oder Polyacrylsäure genannt. Typische Mengen für diese Substanzen sind 0,1 bis 2 Gewichtsprozent.

Ein besonders geeigneter Komplexbildner ist insbesondere Ethylendiamintetraessigsäure, kurz EDTA, respektive Salze davon, wie Natriumsalze. Typische Mengen für diese Substanzen sind 0,01 bis 1 Gewichtsprozent.

Die erfindungsgemässen Kontaktlinsenpflegemittel sind für alle Arten von Kontaktlinsen geeignet. Darunter fallen insbesondere die sogenannten harten und weichen Kontaktlinsen, aber auch die sogenannten hart-flexiblen oder hoch-gasdurchlässigen Kontaktlinsen. Die erfindungsgemässen Kontaktlinsenpflegemittel weisen eine antimikrobielle Wirkung und darüber hinaus eine reinigende Wirkung auf. Je nach speziell beabsichtigtem Verwendungszweck können die erfindungsgemässen Kontaktlinsenpflegemittel als Reinigungsmittel, als Desinfektionsmittel, oder z.B. als Lösung zur Aufbewahrung, zum Abspülen, Befeuchten oder Einweichen von Kontaktlinsen eingesetzt werden. Alle diese Lösungen zeichnen sich durch eine gute Reinigungs- und Desinfektionswirkung bei hoher Verträglichkeit in einer einzigen Lösung aus.

Ferner weisen die erFndungsgemässen Kontaktlinsenpflegemittel gegenüber aus dem Stand der Technik bekannten Pflegemitteln, z.B. gegenüber der Bausch & Lomb Kombilösung, bei besserer antimikrobieller Wirksamkeit überraschenderweise ein deutlich besseres Cytotoxizitätsverhalten auf. Das Gesamtspektrum der Eigenschaften der erfindungsgemässen Kontaktlinsenpflegemittel wird daher gegenüber den Eigenschaften von Kontaktlinsenpflegemitteln, die aus dem Stand der Technik bekannt sind, als deutlich positiver eingeschätzt.

Die erfindungsgemässen Kontaktlinsenpflegemittel werden auf an sich bekannte Weise hergestellt, insbesondere durch konventionelles Vermischen der Bestandteile mit Wasser bzw. Lösen der Bestandteile in Wasser.

Die erfindungsgemässen Zusammensetzungen sind insbesondere zur Reinigung und Desinfektion von Kontaktlinsen geeignet. Die erfindungsgemässen Kontaktlinsenpflegemittel werden auf an sich bekannte Art und Weise verwendet, z.B. indem eine Kontaktlinse mit dem Kontaktlinsenpflegemittel für eine Zeitspanne in Kontakt gebracht wird, die für eine Reinigung oder Desinfektion ausreichend ist. Je nach Linsentyp und Verschmutzungsgrad ist hierfür eine Zeitspanne von einigen Minuten bis zu etwa 24 Stunden, bevorzugt bis zu etwa 4 bis 12 Stunden, ausreichend.

Eine bevorzugte erfindungsgemässe Lösung enthält beispielsweise

| | |
|---|---|
| Biguanid | 0,0005 bis 0,05 mg/ml |
| 2-Amino-2-hydroxymethyl-1,3-propandiol | 0,67 bis 4,03 mg/ml |
| 2-Amino-2-hydroxymethyl-1,3-propandiol . HCl | 2,64 bis 4,02 mg/ml, |

wobei die Gesamtmenge Puffer geringer als 6 mg/ml ist,
ferner kann sie vorzugsweise enthalten:

| | |
|---|---|
| NaCl oder KCI | 3 bis 9 mg/ml |
| | insbesondere 5,5 bis 9 mg/ml |
| oberflächenaktive Substanz | 5 bis 30 mg/ml |
| EDTA | 0,1 bis 2 mg/ml. |

Eine ebenfalls bevorzugte erfindungsgemässe Lösung enthält beispielsweise

| | |
|---|---|
| Biguanid | 0,0005 bis 0,05 mg/ml |
| 2-Amino-2-hydroxymethyl-1,3-propandiol | 1,00 bis 4,00 mg/ml |
| | insbesondere etwa 2,5 mg/ml |

sowie geringe Mengen HCl, um den gewünschten pH-Bereich einzustellen.

Der gewünschte pH-Bereich der erfindungsgemässen Zusammensetzungen liegt insbesondere bei etwa 7,0 bis 7,5, bevorzugt bei 7,1 bis 7,4 und speziell bevorzugt bei 7,3.

Die nachfolgenden Beispiele dienen der Erläuterung der Erfindung. Sie sollen den Gegenstand der Erfindung jedoch in keiner Weise einschränken, insbesondere nicht auf den Gegenstand der Beispiele.

### Vergleichsbeispiel : Antimikrobielle Wirksamkeit

Die Bausch & Lomb Renu Lösung, enthaltend ein Biguanid (0,5 ppm) und einen Borat-Puffer wurde gegenüber den folgenden Testkeimen untersucht: Escherichia coli, Staphylococcus aureus, Pseudomonas aeruginosa, Candida albicans, Aspergillus niger. In der folgenden Tabelle wird das Anfangs-Inokulum angegeben sowie die Keimzahl, die nach 4 bzw. 6 Stunden Einwirkungszeit der Renu Lösung noch festzustellen war:

| Testkeim | Anfangs-Inokulum | Keimzahl | Keimzahl |
|---|---|---|---|
| | | nach 4 h | nach 6 h |
| Escherichia coli | 6,8 . 10⁵ | 9,5 . 10¹ | 4,3 . 10¹ |
| Staphylococcus aureus | 8,9 . 10⁵ | 5,7 . 10¹ | 3,8 . 10¹ |
| Pseudomonas aeruginosa | 1,0 . 10⁶ | 0 | 0 |
| Candida albicans | 1,1 . 10⁶ | 4,9 . 10⁵ | 6,2 . 10⁵ |
| Aspergillus niger | 1,1 . 10⁶ | 5,5 . 10⁵ | 4,3 . 10⁵ |

### Vergleichsbeispiel: Cytotoxizitätstest

Um das cytotoxische Potential zu bestimmen, wurde die Kombi-Lösung von Bausch & Lomb, dem Wachstums-lnhibierungs-Test (Growth Inhibition Test) unterworfen. In diesem Test wird die Abnahme von Zellwachstum in Gegenwart toxischer Substanzen bestimmt, indem der Proteingehalt von behandelten Zellkulturen mit dem Proteingehalt von unbehandelten Zellkulturen nach 72 Stunden Inkubation verglichen wird. Die Testlösungen werden seriell mit dem Zell-Kulturmedium verdünnt (DMEM-FCS). L 929 Zellkulturen werden 72 Stunden in Gegenwart der verschieden stark konzentrierten Lösungen inkubiert. Dabei ergibt sich, dass die Kombi-Lösung bereits bei Konzentrationen von 5 % v/v cytotoxische Effekte induziert.

In dem durchgeführten Test gilt, dass der Proteingehalt ein Mass für das Zellwachstum ist bzw. für die Wachstumsinhibierung, die durch toxische Substanzen induziert wird. Eine Wachstumsinhibierung von mehr als 30 % im Vergleich zu unbehandelten Kulturen wird dabei als klarer cyctotoxischer Effekt betrachtet.

### Kombi-Lösung von Bausch & Lomb

| Konzentration | | | | | | |
|---|---|---|---|---|---|---|
| der Lösung [% v/v] | 20,0 | 10,0 | 5,0 | 2,5 | 1,3 | 0,6 |
| Wachstumsinhibierung [%] | 92 | 68 | 40 | 25 | 11 | 7 |

Diese Ergebnisse belegen, dass die Kombi-Lösung bereits bei 5 % v/v, cytotoxische Effekte induziert.

### Beispiel 1: Formulierung für harte Kontaktlinsen

| 1 ml Lösung enthält: | |
|---|---|
| Biguanid (Cosmocil® ) | 0,002 mg |
| 2-Amino-2-hydroxymethyl-1,3-propandiol | 2,5 mg |
| NaCl | 7,5 mg |
| Poloxamer 407 | 10 mg |
| Hydroxyethylcellulose | 3,2 mg |
| EDTA | 1 mg |

HCl zur pH-Einstellung.

### Beispiel 2: Formulierung für weiche Kontaktlinsen

| 1 ml Lösung enthält: | |
|---|---|
| Biguanid (Cosmocil® ) | 0,001 mg |
| 2-Amino-2-hydroxymethyl-1,3-propandiol | 2,45 mg |
| NaCl | 7,4 mg |
| Poloxamer 407 | 1,0 mg |
| EDTA | 0,25 mg |

HCl zur pH-Einstellung.

### Beispiel 3: Antimikrobielle Wirksamkeit

Die Formulierung gemäss Beispiel 1 wurde gegenüber den folgenden Testkeimen untersucht: Escherichia coli, Staphylococcus aureus, Pseudomonas aeruginosa, Candida albicans, Aspergillus niger. In der folgenden Tabelle wird das Anfangs-Inokulum angegeben sowie die Keimzahl, die nach 6 Stunden Einwirkungszeit der Formulierung gemäss Beispiel 1 noch festzustellen war:

| Testkeim | Anfangs-Inokulum | Keimzahl |
|---|---|---|
| | | nach 6 h |
| Escherichia coli | 1,1 . 10⁶ | 0 |
| Staphylococcus aureus | 1,4 . 10⁶ | 0 |
| Pseudomonas aeruginosa | 1,3 . 10⁶ | 0 |
| Candida albicans | 1,2 . 10⁶ | 4,1 . 10⁵ |
| Aspergillus niger | 5,7 . 10⁵ | 2,8 . 10⁵ |

### Beispiel 4: Cytotoxizitätstest

Analog zum Vergleichsbeispiel wurde die Formulierung gemäss Beispiel 1 dem Wachstums-Inhibierungs-Test (Growth Inhibition Test) unterworfen. Dabei ergibt sich, dass die erfindungsgemässe Lösung selbst bei Konzentrationen von 20 % v/v nur äusserst geringe cytotoxische Effekte induziert, während die Kombi-Lösung bereits bei Konzentrationen von 5 % v/v deutliche cytotoxische Effekte induziert (siehe Vergleichsbeispiel).

| Lösung gemäss Beispiel 1 | | | | | | |
|---|---|---|---|---|---|---|
| Konzentration | | | | | | |
| der Lösung [% v/v] | 20,0 | 10,0 | 5,0 | 2,5 | 1,3 | 0,6 |
| Wachstumsinhibierung [%] | 12 | 5 | 0 | 0 | 0 | 3 |

## Patentansprüche

1. Kontaktlinsenpflegemittel enthaltend ein Biguanid der Formel 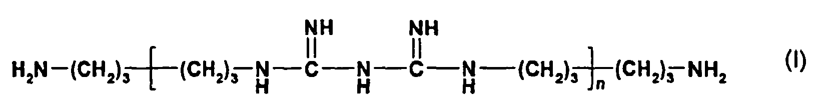 oder ein Salz davon, worin n eine ganze Zahl zwischen 1 und 500 bedeutet, **dadurch gekennzeichnet, dass** es als Puffer 0,05 bis weniger als 0,6 Gewichtsprozent einer Verbindung der Formel 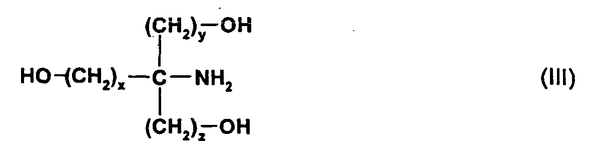 oder ein Salz davon enthält, worin x, y und z, unabhängig voneinander, eine ganze Zahl, mindestens 1, bedeuten und die Summe aus x, y und z 3 bis 9 beträgt.

2. Kontaktlinsenpflegemittel gemäss Anspruch 1, worin das Biguanid ein Gemisch einer Verbindung der Formel I oder eines Salzes davon, 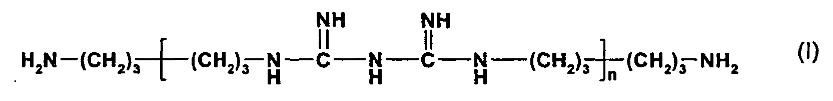 worin n eine ganze Zahl zwischen 1 und 500 bedeutet, mit einer Verbindung der Formel II oder eines Salzes davon ist, 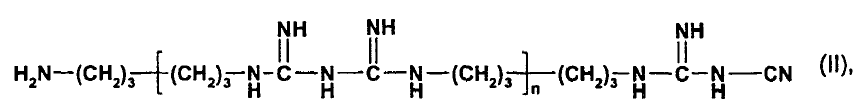 worin ebenfalls n eine ganze Zahl zwischen 1 und 500 bedeutet.

3. Kontaktlinsenpflegemittel gemäss Anspruch 1, worin das Biguanid eine Verbindung der Formel I ist, 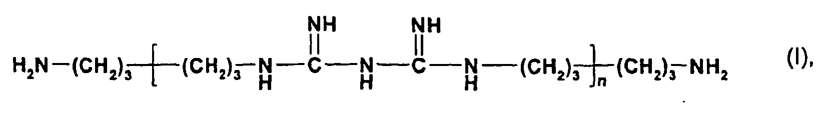 worin n eine ganze Zahl zwischen 1 und 500 bedeutet, oder ein Salz einer Verbindung der Formel I und worin der Puffer Trometamol ist oder ein Salz davon.

4. Kontaktlinsenpflegemittel gemäss Anspruch 1 enthaltend als Puffer 0,1 bis weniger als 0,6 Gewichtsprozent einer Verbindung der Formel (III) oder ein Salz davon.

5. Kontaktlinsenpflegemittel gemäss Anspruch 1 enthaltend
| | |
|---|---|
| Biguanid | 0,0005 bis 0,05 mg/ml |
| 2-Amino-2-hydroxymethyl-1,3-propandiol | 1,00 bis 4,00 mg/ml |
| | insbesondere etwa 2,5 mg/ml |
und HCl zur Einstellung eines pH-Wertes von 7,0 bis 7,5.

6. Kontaktlinsenpflegemittel gemäss Anspruch 1 enthaltend zusätzlich einen oder mehrere Zusatzstoffe ausgewählt unter Substanzen, die die Tonizität beeinflussen, oberflächenaktiven Substanzen, die Viskosität beeinflussenden Substanzen und Komplexbildnern.

7. Kontaktlinsenpflegemittel gemäss Anspruch 1 enthaltend
| | |
|---|---|
| Biguanid | 0,002 mg/ml |
| 2-Amino-2-hydroxymethyl-1,3-propandiol | 2,5 mg/ml |
| NaCl | 7,5 mg/ml |
| Poloxamer 407 | 10 mg/ml |
| Hydroxyethylcellulose | 3,2 mg/ml |
| EDTA | 1 mg/ml |
und HCl zur pH-Einstellung.

8. Kontaktlinsenpflegemittel gemäss Anspruch 1 enthaltend
| | |
|---|---|
| Biguanid | 0,001 mg/ml |
| 2-Amino-2-hydroxymethyl-1,3-propandiol | 2,45 mg/ml |
| NaCl | 7,4 mg/ml |
| Poloxamer 407 | 1,0 mg/ml |
| EDTA | 0,25 mg/ml |
und HCl zur pH-Einstellung.

9. Verwendung eines Kontaktlinsenpflegemittels gemäss Anspruch 1 zur Reinigung und/oder Desinfektion einer Kontaktlinse.

10. Verfahren zur Reinigung und/oder Desinfektion einer Kontaktlinse, **dadurch gekennzeichnet, dass** ein Kontaktlinsenpflegemittel gemäss Anspruch 1 für eine Zeitspanne mit einer Kontaktlinse in Kontakt gebracht wird, die für eine Reinigung oder Desinfektion ausreichend ist.

## Claims

1. Contact lens care system comprising a biguanide of formula 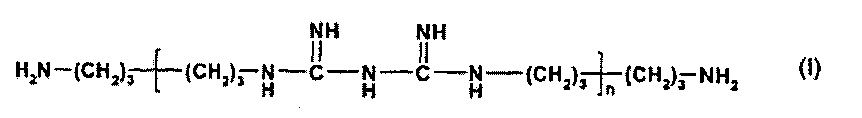 or a salt thereof, wherein n is an integer from 1 to 500, **characterised in that** it comprises as buffer 0.05 to less than 0.6% by weight of a compound of formula 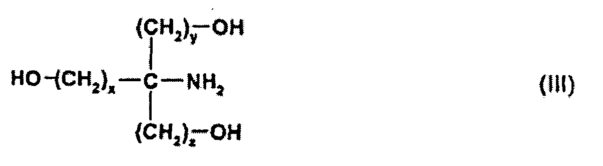 or a salt thereof, wherein x, y and z, independently of one another, are a whole number, at least 1, and the sum of x, y and z is 3 to 9.

2. Contact lens care system according to claim 1, wherein the biguanide is a mixture of a compound of formula I or a salt thereof, 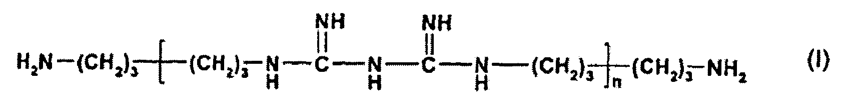 wherein n is an integer from 1 to 500, with a compound of formula II or a salt thereof, 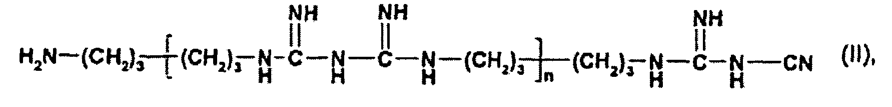 wherein n is likewise an integer from 1 to 500.

3. Contact lens care system according to claim 1, wherein the biguanide is a compound of formula I 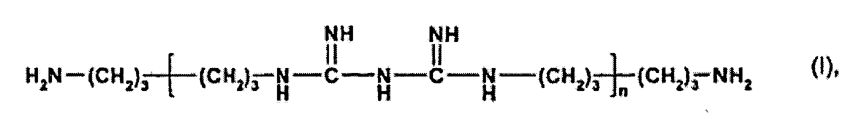 wherein n is an integer from 1 to 500, or a salt of a compound of formula I, and wherein the buffer is trometamol or a salt thereof.

4. Contact lens care system according to claim 1, comprising as buffer from 0.1 to less than 0.6% by weight of a compound of formula (III) or a salt thereof.

5. Contact lens care system according to claim 1, comprising
| | |
|---|---|
| biguanide | 0.0005 to 0.05 mg/ml |
| 2-amino-2-hydroxymethyl-1,3-propanediol | 1.00 to 4.00 mg/ml |
| | especially ca. 2.5 mg/ml |
and HCl to establish a pH value of 7.0 to 7.5.

6. Contact lens care system according to claim 1, comprising in addition one or more additives selected from substances that influence tonicity, surface-active substances, compounds that influence viscosity and complex formers.

7. Contact lens care system according to claim 1, comprising
| | |
|---|---|
| biguanide | 0.002 mg/ml |
| 2-amino-2-hydroxymethyl-1,3-propanediol | 2.5 mg/ml |
| NaCl | 7.5 mg/ml |
| poloxamer 407 | 10 mg/ml |
| hydroxyethylcellulose | 3.2 mg/ml |
| EDTA | 1 mg/ml |
and HCl to establish pH.

8. Contact lens care system according to claim 1, comprising
| | |
|---|---|
| biguanide | 0.001 mg/ml |
| 2-amino-2-hydroxymethyl-1,3-propanediol | 2.45 mg/ml |
| NaCl | 7.4 mg/ml |
| poloxamer 407 | 1.0 mg/ml |
| EDTA | 0.25 mg/ml |
and HCl to establish pH.

9. Use of a contact lens care system according to claim 1 for cleaning and/or disinfecting a contact lens.

10. Method of cleaning and/or disinfecting a contact lens, **characterised in that** a contact lens care system according to claim 1 is brought into contact with a contact lens for a period of time sufficient for cleaning or disinfection to take place.

## Revendications

1. Produit pour l'entretien des lentilles de contact contenant un biguanide de formule 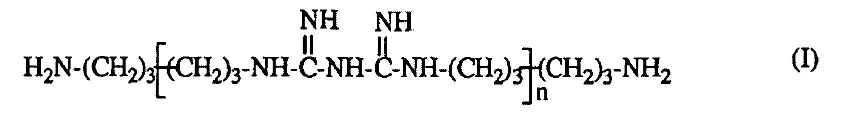 ou un de ses sels, où n est un nombre entier de 1 à 500, **caractérisé en ce qu'**il contient en tant que tampon 0,05 à moins de 0,6 pour-cent en poids d'un composé de formule 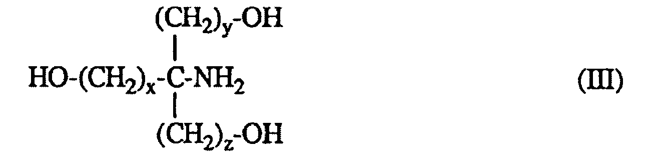 ou d'un de ses sels, les indices x, y et z représentant chacun indépendamment des autres un nombre entier valant au moins 1, et la somme de x, de y et de z valant 3 à 9.

2. Produit pour l'entretien des lentilles de contact selon la revendication 1, dans lequel le biguanide est un mélange d'un composé de formule I ou d'un de ses sels, 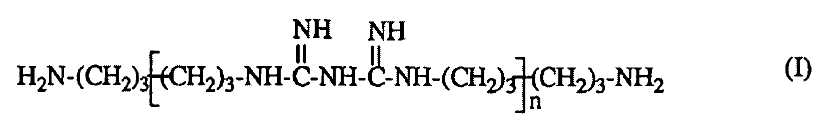 dans laquelle n est un nombre entier de 1 à 500, et d'un composé de formule II ou d'un de ses sels 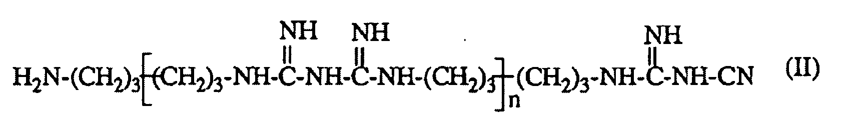 dans laquelle n aussi est un nombre entier compris entre 1 et 500.

3. Produit pour l'entretien des lentilles de contact selon la revendication 1, dans lequel le biguanide est un composé de formule 1 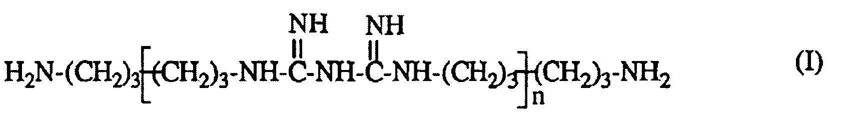 dans laquelle n est un nombre entier de 1 à 500, ou un sel d'un composé de formule I, et où le tampon est le trométamol ou un sel de ce dernier.

4. Produit pour l'entretien des lentilles de contact selon la revendication 1, contenant en tant que tampon 0,1 à moins de 0,6 pour-cent en poids d'un composé de formule III ou d'un de ses sels.

5. Produit pour l'entretien des lentilles de contact selon la revendication 1, contenant :
| | |
|---|---|
| Biguanide | 0,0005 à 0,05 mg/ml |
| 2-amino-2-hydroxyméthyl-1,3-propanediol | 1,00 à 4,00 mg/ml, en |
| | particulier environ 2,5 mg/ml |
et du HCl pour ajuster le pH à une valeur de 7,0 à 7,5.

6. Produit pour l'entretien des lentilles de contact selon la revendication 1, qui contient en outre un ou plusieurs additifs choisis parmi des substances qui influent sur la tonicité, des substances tensioactives, des substances qui agissent sur la viscosité et des agents complexants.

7. Produit pour l'entretien des lentilles de contact selon la revendication 1, contenant :
| | |
|---|---|
| Biguanide | 0,002 mg/ml |
| 2-amino-2-hydroxyméthyl-1,3-propanediol | 2,5 mg/ml |
| NaCl | 7,5 mg/ml |
| Poloxamer 407 | 10 mg/ml |
| Hydroxyéthylcellulose | 3,2 mg/ml |
| EDTA | 1 mg/ml |
et du HCl pour ajuster le pH.

8. Produit pour l'entretien des lentilles de contact selon la revendication 1, contenant :
| | |
|---|---|
| Biguanide | 0,001 mg/ml |
| 2-amino-2-hydroxyméthyl-1,3-propanediol | 2,45 mg/ml |
| NaCl | 7,4 mg/ml |
| Poloxamer 407 | 1,0 mg/ml |
| EDTA | 0,25 mg/ml |
et du HCl pour ajuster le pH

9. Utilisation d'un produit pour l'entretien des lentilles de contact selon la revendication 1 pour nettoyer et/ou désinfecter une lentille de contact.

10. Procédé pour nettoyer et/ou désinfecter une lentille de contact, **caractérisé en ce qu'**on met en contact avec une lentille de contact un produit pour l'entretien des lentilles de contact selon la revendication 1 pendant un laps de temps suffisant pour un nettoyage ou une désinfection.
